# EUROPEAN PATENT APPLICATION

(11) **EP 1 607 483 A1**
(43) Date of publication of application: **21.12.2005**
(21) Application number: 04718249.8
(22) Date of filing: 08.03.2004
(51) Int. Cl.: C12N 15/861, C12N 15/31, A61K 48/00, A61P 35/00

(54) **A RECOMBINANT CONSTRUCTED BY A VIRUS VECTOR AND A HUMAN TUMOR SUPPRESSOR GENE AND ITS USE**

(30) Priority: 06.03.2003 CN 03165032
(71) Applicant: Peng, Zhaohui, c/o SiBiono Co., Shenzhen, Guangdong 518057 (CN); Zhang, Xiaozhi, Shenzhen, Guangdong 518057 (CN)
(72) Inventor: Peng, Zhaohui, c/o SiBiono Co., Shenzhen, Guangdong 518057 (CN); Zhang, Xiaozhi, Shenzhen, Guangdong 518057 (CN)
(74) Representative: Grünecker, Kinkeldey, Stockmair & Schwanhäusser Anwaltssozietät
(86) International application number: PCT/CN2004/000465
(87) International publication number: WO 2004/078987

(57) **Abstract**

The invention relates to a recombinant virus, which is engineered to contain a human tumor suppressor gene, and its application. This recombinant constructed by DNA cloning technology is combined with virus vector and human tumor suppressor gene expression cassette, which can amplify and propagate in specific genetic-engineered cell lines, and also can express tumor suppressor protein in eukaryote cells. In this invention, the recombinant adenovirus-p53 DNA is constructed with adenoviral vector and human p53 gene by homologous recombination in prokaryotic cells (*E*. *coli*). The human p53 gene expression cassette is the specific sequence, which is composed of promoter-p53 cDNA-poly (A). The recombinant p53 adenovirus of this invention can be prepared as clinical gene therapy products, which apply in treating and preventing human malignant tumors.

## Description

### Field of the Invention

The present invention relates to genetic engineering technology, especially to a recombination of human tumor suppressor gene p53 and adenovirus vector.

### Background of the Invention

As the development of molecular biological technology, especially the improvement of genetic engineering technology, gene therapies for malignant tumors have become the hot research topic. Nowadays, there are more than 600 gene therapy programs permitted in clinical experiments. Some of these therapies showed positive results indicating promising prospective.

The vectors used in gene therapy could not be used to treat the diseases directly. On the other hand, the genes which could be used to treat the diseases only have potential treating capability, since it is very difficult to directly enter and then express protein in the target cells. In order to make the potential therapeutic genes take effect, the vector should first be combined with the target gene, then carry the gene into the target cells by transfection, and finally the target gene could enter and express protein in the cells. Therefore, the key of gene therapy is to construct the recombinant DNA for the therapeutic gene and the gene vector.

The common way to recombine the target gene expression cassette with its vector is homologous recombination in eukaryotic cells, which is a very complicated and tedious process. However, using prokaryotic cells for homologous recombination and constructing recombinant vectors could solve the above problems.

A bottle-neck in tumor gene therapy lies in the lack of specific, targeting and efficient gene transfer vectors. At present, there are two kinds of vectors used in gene therapy research including viral vectors and non-viral vectors. The common viral vectors include adenovirus vectors, adeno-associated virus vectors and -retrovirus vectors. Adenovirus vector is the most common. Its advantages include high transfection rate, relative safety and ease of operability, the ability to carry large gene fragments and the ability to prepare high titer viral particles, suitable for industrial production, and the ability to infect cells not only in division phase but also in non-division phase. However, its disadvantages include a lack of target-specific infection and production of immunogenicity . Therefore, it is necessary to improve adenovirus vector for gene therapy. Research indicates that a foreign gene carried by adenovirus vector that is E1 and E3 region deleted could lead to long-term protein expression and the immunogenicity reduction. The retrovirus vector could carry a foreign gene and integrate into the target cells' genome, thus realizing the stable and lasting gene expressions. However, the retrovirus has the following disadvantages: low reproduction titer *in vitro,* low transfection efficiency, infecting only the cells in division phase, and random recombination with chromosomes having potentially carcinogenic activity. Other viral or non-viral vectors used in gene transformations also have different advantages and disadvantages.

### Summary of the Invention

The object of this invention is to recombine the potentially therapeutic genes with their vectors, thus providing a recombinant of a viral vector and a human tumor suppressor gene. This recombinant can amplify and propagate in special genetic-engineered cells and express protein directly in eukaryotic cells and the recombinant could then be used to prevent and/or treat tumors.

The object of this invention is also to provide a method for producing this recombination and its application in preparing medicines for tumor prevention and treatment.

This invention provides a recombinant of a viral vector and a human tumor suppressor gene. This recombinant is constructed with a viral vector and a human tumor suppressor gene expression cassette by DNA cloning technology. The resulting product is the recombinant, which can amplify and propagate in specific genetically engineered cells and also can express tumor suppressor protein in eukaryotic cells.

The recombinant vector can be either DNA virus or RNA virus. The preferred vector is adenovirus vector or combined vector containing adenovirus vector sequence. The most preferable vector is the adenovirus vector.

The human tumor suppressor gene can be any tumor suppressor genes, the most preferable one is p53.

The recombinant combined with adenovirus vector and p53 gene is defined as recombinant p53 adenovirus, which has the following sequence: 
the right end of adenovirus
5-ATGTTTACCGCCACACTCGCAGGGTCTGCACCTGGTGCGGG
TCTCATCGTACCTCAGCACCTTCCAGATC70TCTGACATGCGATGT
CGACTCGACTGCTTCGCGATGTACGGGCCAGATATACGCGTATCT
GAGGGGACTAGGGTGTGTTTAGGCGAAAAGCGGGGCTTCGGTTG
TACGCGGTTAGGAGTCCCCTCAGGATATAGTAGTTTCGCTTTTGC
ATAGGGAGGGGGAAATGTAGTCTTATGCAATACTCTTGTAGTCTT
GCAACATGGTAACGATGAGTTAGCAACATGCCTTACAAGGAGAGA
AAAAGCACCGTGCATGCCGATTGGTGGAAGTAAGGTGGTACGAT
CGTGCCTTATTAGGAAGGCAACAGACGGGTCTGACATGGATTGGA
CGAACCACTGAATTCCGCATTGCAGAGATATTGTATTTAAGTGCCT
AGCTCGATACAATAAACGCCATTTGACCATTCACCACATTGGTGTG
CACCTCCAAGCTTGGTACCGAGCTCGGATCCCG523CTAGAGCCAC
CGTCCAGGGAGCAGGTAGCTGCTGGGCTCCGGGGACACTTTGCG
TTCGGGCTGGGAGCGTCTTTCCACGACGGTGACACGCTTCCCTG
GATTGGCAGCCAGACTGCTTTCCGGGTCACTGCC655ATGGAGGAG
CCGCAGTCAGATCCTAGCGTCGAGCCCCCTCTGAGTCAGGAAAC
ATTTTCAGACCTATGGAAACTACTTCCTGAAAACAACGTTCTGTCC
CCCTTGCCGTCCCAAGCAATGGATGATTTGATGCTGTCCCCGGAC
GATATTGAACAATGGTTCACTGAAGACCCAGGTCCAGATGAAGCT
CCCAGAATGCCAGAGGCTGCTCCCCCCGTGGCCCCTGCACCAGC
AGCTCCTACACCGGCGGCCCCTGCACCAGCCCCCTCCTGGCCCC
TGTCATCTTCTGTCCCTTCCCAGAAAACCTACCAGGGCAGCTACG
GTTTCCGTCTGGGCTTCTTGCATTCTGGGACAGCCAAGTCTGTGA
CTTGCACGTACTCCCCTGCCCTCAACAAGATGTTTTGCCAACTGG
CCAAGACCTGCCCTGTGCAGCTGTGGGTTGATTCCACACCCCCG
CCCGGCACCCGCGTCCGCGCCATGGCCATCTACAAGCAGTCACA
 
GCACATGACGGAGGTTGTGAGGCGCTGCCCCCACCATGAGCGCT
GCTCAGATAGCGATGGTCTGGCCCCTCCTCAGCATCTTATCCGAG
TGGAAGGAAATTTGCGTGTGGAGTATTTGGATGACAGAAACACTT
TTCGACATAGTGTGGTGGTGCCCTATGAGCCGCCTGAGGTTGGC
TCTGACTGTACCACCATCCACTACAACTACATGTGTAACAGTTCCT
GCATGGGCGGCATGAACCGGAGGCCCATCCTCACCATCATCACA
CTGGAAGACTCCAGTGGTAATCTACTGGGACGGAACAGCTTTGAG
GTGCGTGTTTGTGCCTGTCCTGGGAGAGACCGGCGCACAGAGGA
AGAGAATCTCCGCAAGAAAGGGGAGCCTCACCACGAGCTGCCCC
CAGGGAGCACTAAGCGAGCACTGCCCAACAACACCAGCTCCTCT
CCCCAGCCAAAGAAGAAACCACTGGATGGAGAATATTTCACCCTT
CAGATCCGTGGGCGTGAGCGCTTCGAGATGTTCCGAGAGCTGAA
TGAGGCCTTGGAACTCAAGGATGCCCAGGCTGGGAAGGAGCCAG
GGGGGAGCAGGGCTCACTCCAGCCACCTGAAGTCCAAAAAGGGT
CAGTCTACCTCCCGCCATAAAAAACTCATGTTCAAGACAGAAGGG
CCTGACTCAGACTGA1837CATTCTCCACTTCTTGTTCCCCACTGACA
GCCTCCCACCCCCATCTCTCCCTCCCCTGCCATTTTGGGTTTTGG
GTCTTTGAACCCTTGCTTGCAATAGGTGTGCGTCAGAAGCACCCA
GGACTTCCATTTGCTTTGTCCCGGGGCTCCACTGAACAAGTTGGC
CTGCACTGGTGTTTTGTTGTGGGGAGGAGGATGGGGAGTAGGAC
ATACCAGCTTAGATTTTAAGGTTTTTACTGTGAGGGATGTTTGGGA
GATGTAAGAAATGTTCTTGCAGTTAAGGGTTAGTTTACAATCAGCC
ACATTCTAGGTAGGGGCCACTTCACCGTACTAACCAGGGAAGCTG
TCCCTCACTGTTGAATTTTCTCTAACTTCAAGGCCCATATCTGTGA
AATGCTGGATTTGCCCTACCTCGGAATGCTGGCATTTGCACCTAC
CTCACAGAGTGCATTGTGAGGGTT2297AATGAAATAATGTACATCT
GGCCTTGAAACCACCTTTTATTACATGGGGTCTAGCGGATCCAC
TAGTAACGCCGCCAGTGTGCTGGAATTCTGCAGATATCCATCACA
CTGGCGGCCGCTCGAGCATGCATCTAGAGCTCGCTGATCAGCCT
CGACTGTGCCTTCTAGTTGCCAGCCATCTGTTGTTTGCCCCTCCC
CCGTGCCTTCCTTGACCCTGGAAGGTGCCACTCCCACTGTCCTTT
CCTAATAAAATGAGGAAATTGCATCGCATTGTCTGAGTAGGTGTCA
 
TTCTATTCTGGGGGGTGGGGTGGGGCAGGACAGCAAGGGGGAG
GATTGGGAAGACAATAGCAGGCATGCTGGGGATGCGGTGGGCTC
TATGGCTTCTGAGGCGGAAAGAACCAGCTGGGGCTCGAGGGGGA
TCCCCACGCTAGAGCT2733GACTATAATAATAAAACGCCAACTTTGA
CCCGGAACGCGGAAAACACCTGAGAAAAACACCTGGGCGAGTCT
CCACGTAAACGGTCAAAGTCCCCGCGGCCCTAGACAAATATTA
2848- the left end of adenovirus 5
 In which:
1. the right end of adenovirus 5 and the left end of adenovirus 5 are described in the full sequence of adenovirus 5 (Genbank No: NC_001406)
2. 1-70: the right arm of adenovirus (the 70^{th} base locates at 3328 of adenovirus 5 gene sequence)
3. 71-523: Rous Sarcoma Virus (RSV) LTR (promoter)
4. 524-655: 5' non-translation region
5. 656-1837: p53 gene coding sequence
6. 1838-2733: 3' non-translation region (poly Adenosine (poly A) tail starting at 2298)
7. 2734-2848: the left arm of adenovirus (base at 2734 is positioned at 452 of adenovirus 5 gene sequence)

The gene expression cassette of the recombinant is a specific sequence composed of promoter-p53 cDNA-poly Adenosine, in which the upstream of the cassette contains any eukaryotic cell promoter, prokaryotic cell promoter or virus promoter, and the downstream contains any eukaryotic cells poly adenosine (polyA).

The recombinant DNA of this invention was obtained as described below. The recombinant virus vector was obtained in prokaryotic cells by homologous recombination. First, the recombinant pGT-2 was constructed by homologous recombination of the adenovirus with plasmid pGT-1 (including the adenovirus's inverted terminal repeats on both ends) in *E.coli*. Second, recombinant pGT-3 was constructed by homologous recombination of pGT-2 with the artificial sequence "the right arm of adenovirus /promoter-p53cDNA-poly adenosine/the left arm of adenovirus " in *E.coli.* Finally, the recombinant p53 adenovirus obtained by discarding the prokaryotic plasmid sequence using endonuclease *P*acl.

In fact, this recombinant can be obtained in any prokaryotic cells by homologous recombination.

According to the above methods, the long terminal repeats (LTR) on both sides of adenovirus were amplified by PCR and *Pac*l restriction enzymatic sites were introduced respectively. Both LTR fragments were cloned into pUC18 vector and produced pGT-1 recombinant sequence. The constructed pGT-1 and adenovirus 5 were then co-transfected into the *E*.*coli* (BJ5183, preserved by SiBiono Company, preserve No.: P-e012). The adenovirus 5 gene was then homologously recombined with pGT-1. The positive virus clone was then amplified, screened by PCR, and tested using restriction enzymes. Finally, recombinant vector pGT-2 containing adenovirus 5 full gene sequence was obtained.

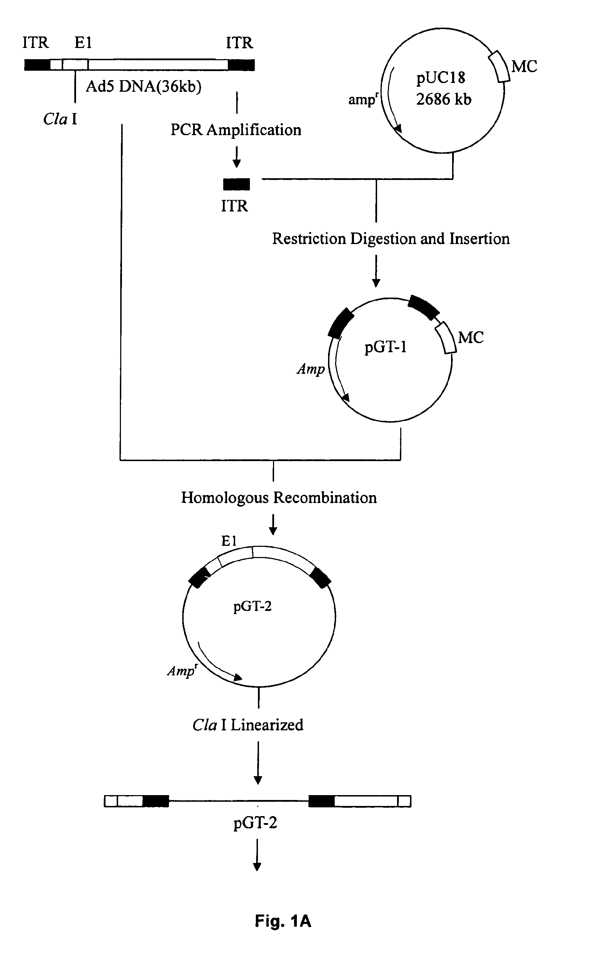

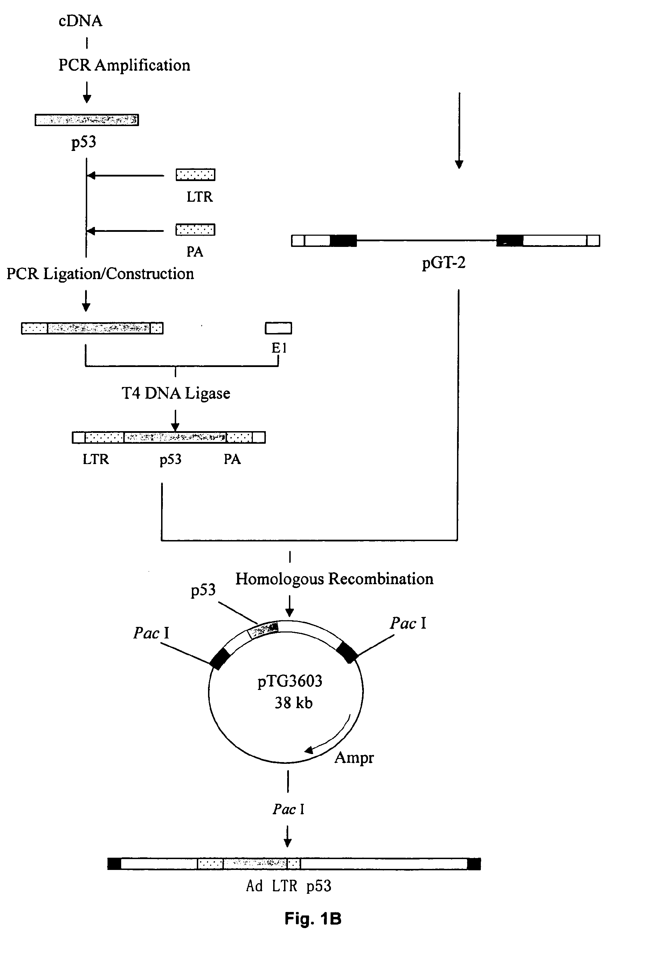

Using 5' ATGGAGGAGCCGCAGTCAGATC and 5'ATATCTGCAGAATTCCA GCAC as primers, human tumor suppressor p53 gene was amplified by PCR. The full sequence of p53 gene (including the 5' and 3' non-translation sequence) was then cloned into vector pUC19 and confirmed by DNA sequencing. Next, the RSV (rous sarcoma virus) LTR sequence (containing promoter sequence) and the PA sequence of BGH and E1 sequence of adenovirus were amplified by PCR. A linker sequence was attached on one side of each aforesaid sequence and confirmed by DNA sequencing. In the next PCR reaction, LTR and PA sequences were attached to the 5' and 3' ends of p53, respectively. The adenovirus E1 region and its upstream sequence were combined to the outer side of p53, and the p53 gene expression cassette was thus constructed (see Figure 1).

The *E.coli* BJ5183 was co-transfected by recombinant vector pGT-2 and p53 gene expression cassette, in which the homologous recombination occurred. The positive clones were then amplified, PCR screened, and confirmed via restriction enzyme reaction. The resulting recombinant vector was pGT-3, which contained most of the adenovirus 5 sequence (its E1 region and part of the upstream sequence were replaced by p53 expression cassette). The recombinant vector pGT-3 was linearized by *Pac*l and the sequence originated from pUC18 was discarded. Then the pGT-3 was used to transfect 293 cells (preserved by SiBiono Company, preservation No.: E-393). The recombinant was packed in cells and produced human tumor suppressor gene p53 containing adenovirus cis-acting sequence and LTR promoter. The resulting recombinant p53 adenovirus had a high transfection rate, was easy to operate and was controlled by a single promoter.

This recombinant p53 adenovirus had the following characteristics:

It was constructed with adenovirus vector and p53 gene artificial expression cassette,
1. Structure: It was a living recombinant adenovirus which was different from other chemical synthetic medicines, herbs and genetically engineered medicines. It was highly biologically active, could be directly expressed *in vivo* and was highly effective in clinical application. Adenovirus could carry large gene fragment and had high transfection rate, which could be prepared as high titer virus particles and had a very broad host range, and proved very safe. The immunogenicity of adenovirus vector was greatly reduced especially after modification, thus the target gene was easily stabilized and expressed *in vivo.* In p53 artificial gene expression cassette, the expression of p53 gene was directly controlled by a single promoter of adenovirus vector, and the polyA tail signal was added, thus an intact expression cassette was constituted.
2. Application: This recombinant p53 adenovirus was a broad spectrum anti-tumor medicine. It could be used to treat many malignant tumors. The phase II clinical trials indicated that it had significant treatment effects on head and neck squamous carcinomas and lung cancer, among others. The recombinant p53 adenovirus was especially effective in preventing tumor recurrence. The phase I clinical trial and 3 years post-surgery observations indicated that this recombinant p53 adenovirus had prevented the post-surgery relapse of the larynx cancer patients as a cancer vaccine.

The recombinant p53 adenovirus of this invention could be made into medicines for treatment of many malignant tumors. And it could be made into medicines for prevention of tumorigenesis and post-surgery relapses of tumors.

This recombinant could also be made into medicines for intravenous injection, artery injection, intratumor injection, intramuscular injection, subcutaneous injection, and injection in pleural effusion or ascites.

The recombinant p53 adenovirus of this invention, was firstly used to transfect the specific genetically engineered cells. Then the cells were grown, concentrated, lysed and purified into clinical-grade recombinant p53 adenovirus anti-tumor injection.

The 293 cells used in this invention (ATCC CRL-1573, 32th generation, bought from ATCC, June 13^{th}, 1997) were screened from human embryonic kidney epithelial cells, which was transformed by Adenovirus 5 (Ad5) DNA and contained 11 % of the genome (including E1 a) from Ad5 5' end. The cells were highly permissive for infection by adenovirus, and also permissive for growth of adenovirus.

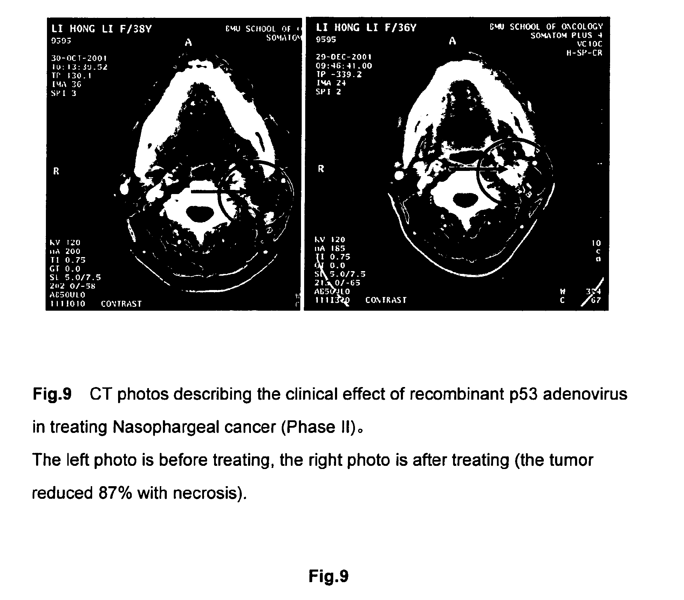

The recombinant p53 adenovirus was applied in phase I clinical trials to 12 larynx cancer patients in middle or advanced stage. The patients were revisited during 31~36 months after treatment. The results indicated that it was very safe to apply the recombinant p53 adenovirus. No relapse was found among these 12 patients. Recently, only 44% of the patients in middle or advanced stage would survive in 3 years, and the recurrence rate was 20% in 6∼12 months after surgery. The clinical trials for this invention indicated that this recombinant not only could be used to treat tumor but also could be used to prevent tumor. The Phase II clinical trials had been carried on ever since 2001. The effect of the treatment was very promising. Results in figure 9~13 indicated that the recombinant of this invention even had effectively treated the patients who were unresponsive to the traditional treatments (chemotherapy and radiotherapy).

The main contribution of this invention lies in taking advantage of the human tumor suppressor gene p53 which could inhibit many tumor cells. The suppressor gene was cloned into E1⁻ adenovirus, which helped p53 gene infect the tumor tissues, and was injected into the tumor. The suppressor gene was then expressed as p53 protein which could inhibit growth of the tumor tissue or even kill the tumor cells. This invention also provided a method to prepare this recombinant p53 adenovirus product, which solved the problem caused by the instability of p53 protein (half-life = 20 minutes) *in vitro.*

The recombinant p53 adenovirus carried the human tumor suppressor gene p53 and expressed it directly in the tumor cells, thus solved the problem that recombinant genetically engineered product (here is p53 protein) could not be made *in vitro.* Using the adenovirus and adeno-associated virus for treatment of tumors, p53 protein could be expressed *in vivo* continuously and highly efficiently. Furthermore, protein modifications in molecular level including phosphorylation, folding, and polymerization were similar to eukaryotic cells. The recombinant p53 adenovirus of this invention could be used to mediate the expression of p53 gene in eukaryotic cells by directly introducing the recombinant to the tumor, effectively using the patient as a source for producing human tumor suppressor factor p53 protein. This method successfully introduced the foreign p53 gene into the human body and allowed it to highly express in the tumor tissue. This has made the gene therapy of tumor and other diseases possible.

### Detailed Description of the Figures

Figure1 show the schematic process of the construction of a recombinant p53 adenovirus.
Figure 2 shows the flow chart of the experimental protocols for the production of the recombinant p53 adenovirus.
Figure 3 shows the result of an agarose gel electrophoresis of a PCR amplification of the recombinant p53 adenovirus after generations of passage, using p53 cDNA as template, and 5'CCACGACGGTGACACGCTTC3', and 5'CAAGCAAGGGTTCAAAGAC3' as primers. This result confirms the stability of the recombinant p53 adenovirus.
Figure 4 shows the result of agarose gel electrophoresis of the PCR amplification of virus DNA obtained from 293 cells which were infected with recombinant p53 adenovirus (preserved by SiBiono Company, preservation No.: No-1, same as the following) 36 hours earlier.
Figure 5 shows the result of Western blot analysis of cell lysate from 293 cells after the cells were infected by recombinant p53 adenovirus for 36 hours.
Figure 6 shows a curve diagram of the killing effect of different dosages of recombinant p53 adenovirus on Hep-2 cells.
Figure 7 shows the curve diagram of growth inhibition effect of recombinant p53 adenovirus on Hep-2 cells.
Figure 8 shows the microscopic photo of Hep-2 cells after the cells were infected with recombinant p53 adenovirus 36 hours earlier.
Figure 9 shows CT photos describing the clinical effect of recombinant p53 adenovirus in treating Nasophargeal cancer (Phase II).
Figure 10 shows CT photos describing the clinical effect of recombinant p53 adenovirus in treating lung cancer (Phase II).
Figure 11 shows CT photos describing the clinical effect of recombinant p53 adenovirus in treating thyroid gland cancer (Phase II).
Figure 12 shows CT photos describing the clinical effect of recombinant p53 adenovirus in treating carcinoma of the cervix (Phase II).
Figure 13 shows CT photos describing the clinical effect of recombinant p53 adenovirus in treating esophagus cancer (Phase II).

### Detailed Description of Embodiments

The following embodiments are further descriptions for this invention. The practice of this invention is not limited to these embodiments.

### Experiment 1:

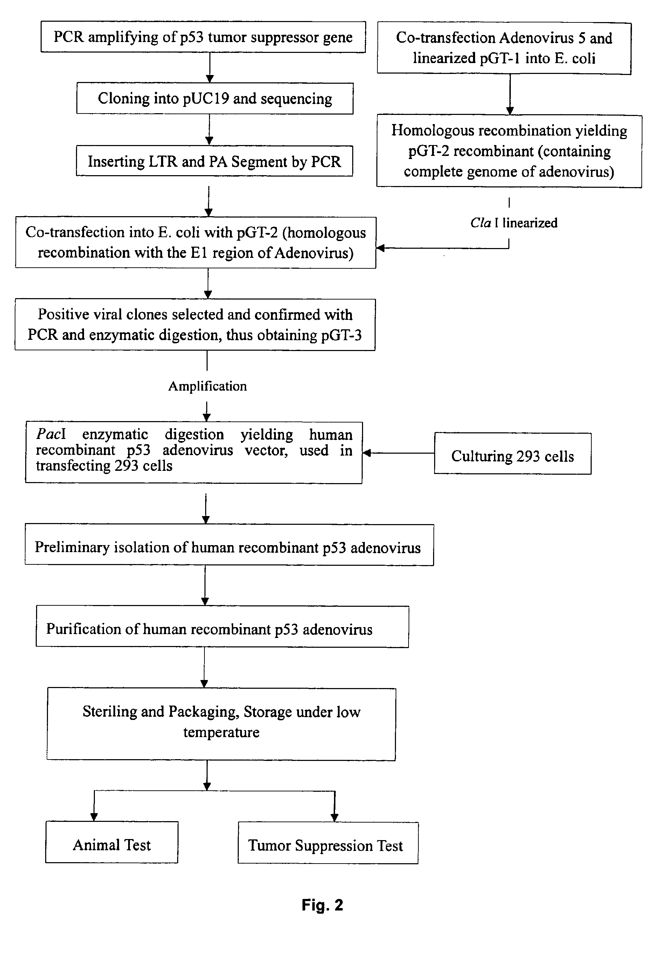

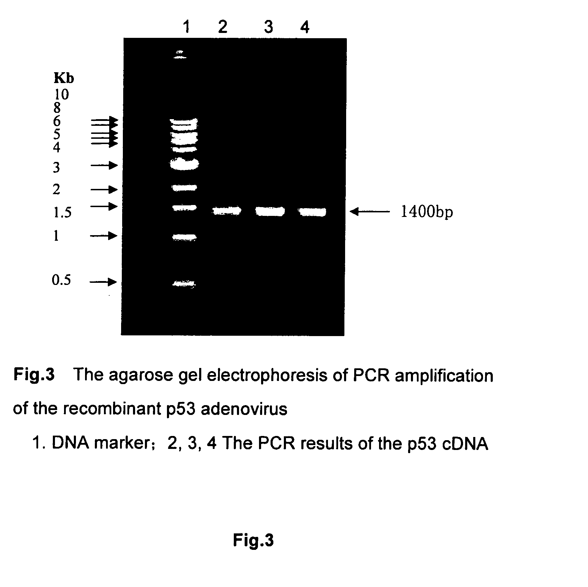

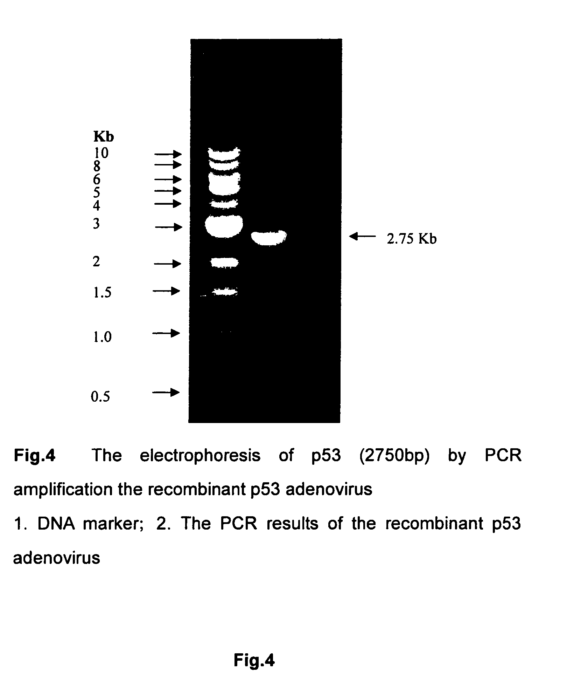

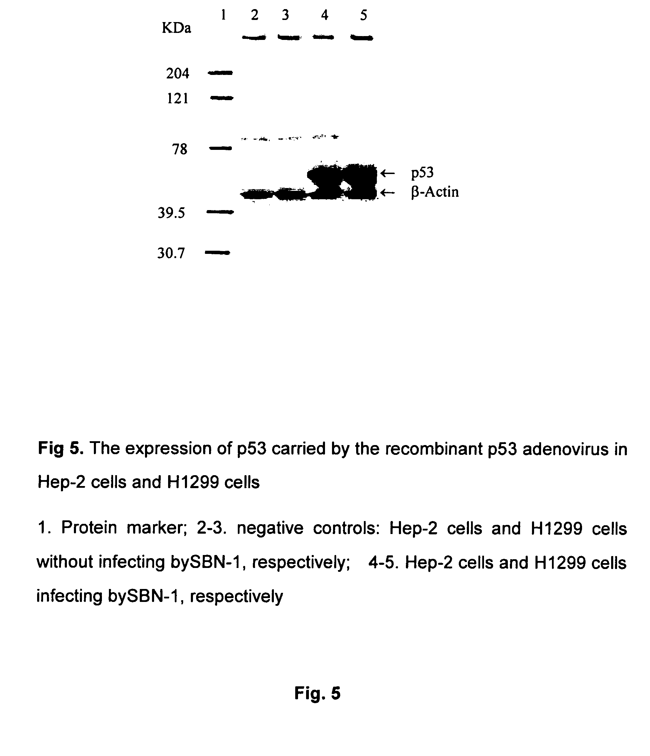

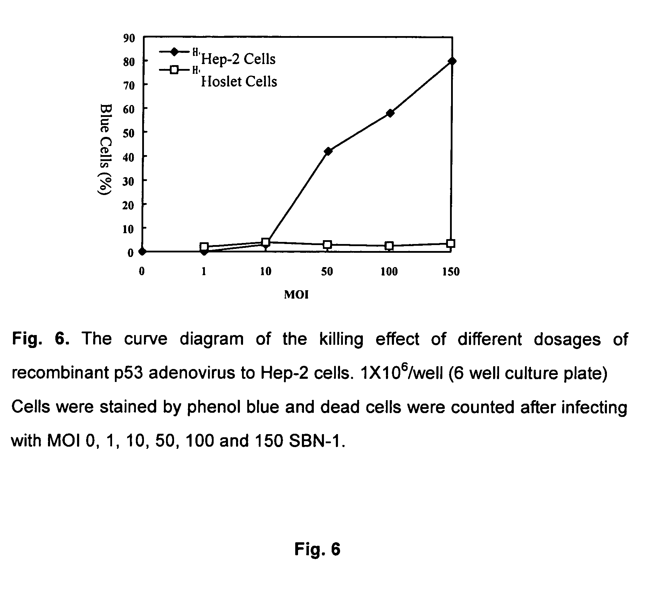

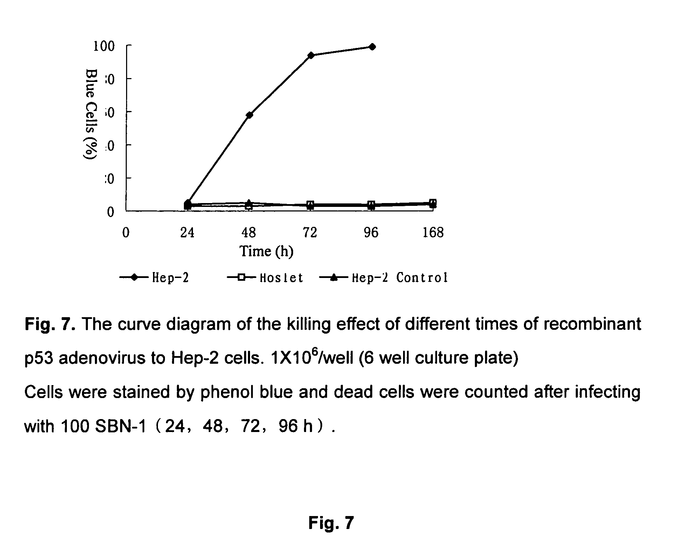

Construction and characterization of the recombinant p53 adenovirus, as described in figure 1 and 2.
1. Two primers were devised according to the published full length sequence of p53 cDNA:
   The two primers were 5' ATGGAGGAGCCGCAGTCAGATC and 5' ATATCTGCAGAATTCCAGCAC. Linker sequences were attached to both ends. Human p53 gene was amplified by PCR reaction using HeLa cell cDNA as a template. The experimental conditions were as follows:
   For the first cycle, denatur DNA for 4 minutes at 94°C, annealing for 1 minute at 58°C, then extend for 2 minutes at 72°C. For each of the rest of the cycles, denature DNA for 1 minute at 94°C, annealing for 1 minute at 58°C, and extend for 2 minutes at 72°C. There were 30 cycles total. Thus a large amount of p53 gene fragments were obtained. The p53 gene was then analyzed using agarose gel electrophoresis. The full sequence of p53 gene was extracted from the gel, purified, cut by restriction enzyme and inserted into pUC19 vector which was cut by the same enzyme. The fragment was then sequenced. The sequence of the coding region tested and the predicted amino acid sequence were the same as the sequence of GenBank Acc XM_058834). Finally the fragment was cleaved by restriction enzyme and recollected.
2. LTR and PA sequences were amplified by PCR. Their primers were respectively: 5′TCTGACATGCGATGTCGACTCG , 5′ CGGCAGTGACCCGGAAAGCAG; 5′ TCACAGAGTGCA TTGTGAGGG, 5′ GCTCTAGCGTGGGGATCCC. Linker sequences were attached to 5' primer and 3' primer, respectively. LTR and PA were amplified by PCR under the same annealing conditions described above. The amplified fragments were purified and confirmed by sequencing.
3. Adenovirus E1 sequence was separately amplified by PCR reaction under the same conditions described above. Enzyme restriction sites for *Bam* HI and Eco RI were respectively linked to primers on both ends. The fragments were tested by sequencing after being amplified.
4. The fragment from step 1 and the two fragments from step 2 were linked by PCR reaction respectively reacted. The experimental conditions were as described above. PCR product LTR-p53-PA was obtained. The resulting sequence was tested by sequencing.
5. The fragment from step 3 and LTR-p53-PA from step 4 were linked by T4 DNA ligase. The resulting sequence was p53 gene cassette.
6. Inverted terminal repeat (IRT) sequences from both ends of adenovirus were amplified by PCR under the same experimental conditions as described above. After being tested by sequencing, the resulting fragments were cloned into pUC18 vector. Thus the recombinant vector pGT-1 was obtained.
7. *E. coli* BJ5183 were co-transfected by recombinant vector pGT-1 and wild adenovirus 5 (ATCC-VR-5, adenovirus 75, titer: 10(6.75) TCID (50)/ml ) DNA. After being kept at 4°C for 30 minutes, the transfected bacteria were treated with heat shock at 42°C for 50 seconds, and then kept at 4°C again for 1 minute. Finally the bacteria were added to 1ml LB media and incubated for 1 hour. The engineered bacteria were then spread on agar medium containing ampicillin and incubated for 24 hours. Single clone was picked by aseptic toothpick and put into a bottle with LB media for 24 hours incubation. Plasmid was extracted by routine methods and screened by *Pac*l enzymatic digestion. The positive clones were pGT-2 (containing the full sequence of Ad5).
8. *E. coli* BJ5183 was co-transfected by recombinant vector pGT-2 and p53 gene cassette. The incubation, screening and characterization method condition was as described above. Positive clones were pGT-3, which contained the adenovirus full gene sequence and inserted p53 gene expression cassette. The vector sequence originated from pUC18 was discarded after the clones were linearized by *Pac*l*.*
9. The positive linear plasmid were purified by CsCl and then used to transfect 293 cells using CaCl₂ method. Cells were collected after 7 days. The cells were centrifuged at 1000rpm for 15 minutes. The supernatant was discarded. Cells were lysed 3 times at 37°C and -80°C. It was again centrifuged at 4000rpm for 30 minutes. Precipitates were discarded. The supernatant infected cells again to amplify the virus which was lysed the same way as described above. The resulting supernatant was density gradient centrifuged with CsCl, 60000rpm at 4°C for 16 hours. The band of recombinant adenovirus was extracted by No. 7 needle. The DNA fragment was added with N1 H buffer and dialyzed at 4°C for 4 hours in a Spectra MW6000 dialysis bag. The DNA solution was sterilized by passing through a 0.25µm filter, then packed and stored at -80°C. Part of the resulting product was used in plaque assay and virus particles titer test.
10. Structure stability test for the recombinant adenovirus. The virus genomic DNA was obtained after several generations of reproduction. The DNA fragments were amplified by PCR using primers from both ends of p53 which were 5'CCACGACGGTGACACGCTTC and 5'CAAGCAAGGGTTCAAAGAC. The results of agarose gel electrophoresis are shown in figure 3. Adenovirus arms at both sides of recombinant p53 adenovirus were devised as primers: 5' TTT CTC AGG TGT TTT CCG C and 5' CAT CGT ACC TCA GCA CCT TC. The result of PCR were shown in figure 4. The results above indicated that the structure of the recombinant p53 adenovirus was stable after many generations of reproduction.
11. The p53 gene expression test in Hep-2 and H1299 cells. 36 hours after being transfected by recombinant p53, the Hep-2 and H1299 cells were lysed by traditional methods. The result of the western blot using p53 protein specific antibody is shown in figure 5.
12. The effects of the time and dosage of recombinant p53 adenovirus on transfecting Hep-2 cells for 36 hours after the recombinant p53 adenovirus infecting Hep-2 cells, the study focused on the different killing effect on Hep-2 cells with different dosages of the recombinant DNA and with different infection times. Cells were stained by phenol blue and dead cells were counted. The results are shown in figure 6 and 7.

### Experiment 2:

The killing effect of recombinant p53 adenovirus on tumor cells: Hep-2 cells were transfected by recombinant p53 adenovirus by common methods.

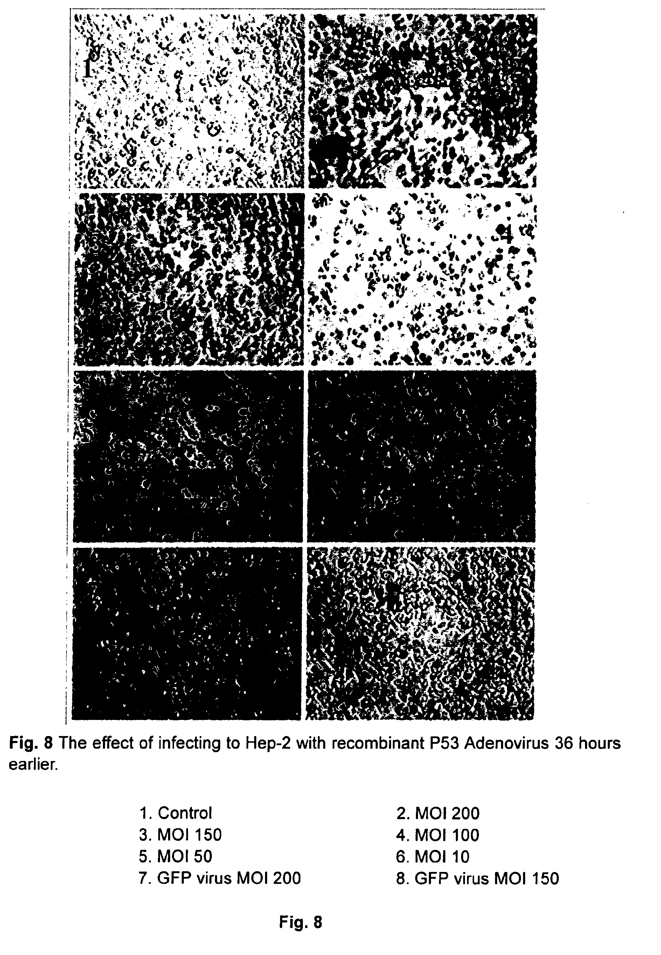

The cell counts of each group were the same. The cells were divided into the following groups: 1. blank control group; 2. MOI 200; 3. MOI 150; 4. MOI 100; 5. MOI 50; 6. MOI 10; 7. GFP viurs MOI 200; 8. GFP virus MOI 150. The cells were incubated for 36 hours after transfection. The killing effect of recombinant p53 adenovirus on tumor cells could be observed under the microscope while MOI was larger than 50. The effects increased with the dosage. The cells were observed shrinking and dying under microscope. The results are shown in figure 8.

### Experiment 3:

### The clinical effect (Nasophargeal cancer) of recombinant p53 adenovirus:

Figure 9 indicates that the recombinant p53 adenovirus was applied in clinical trials in the specific hospitals which was assigned by the State Food and Drug Administration. It also showed CT photos of a Nasophargeal cancer patient before and after treatment of recombinant p53 adenovirus. The photo on the left shows the tumor before treatment, and photo on the right shows the shrunken tumor after treatment (by 87%, and necrosis appeared in tumor tissues). There had been more than 60 patients that received the phase II treatment in clinical trials. The clinical treatment effects were significant.

### Experiment 4:

### The clinical effects (lung cancer) of recombinant p53 adenovirus:

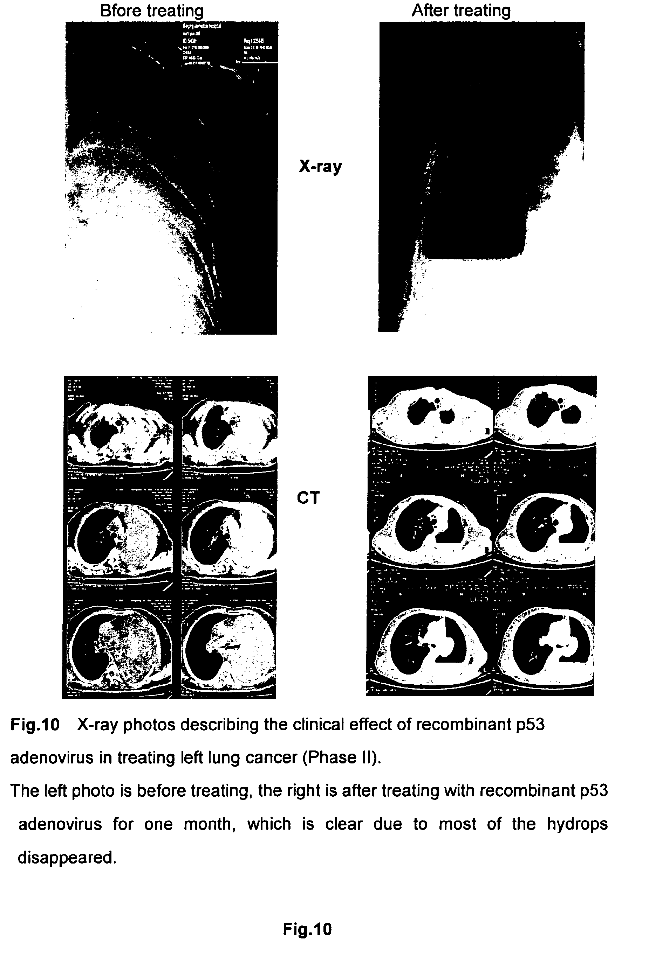

Figure 10 shows the treatment effects of the recombinant p53 adenovirus on lung cancer in phase II clinical trials. A female patient of this experiment had adenocarcinoma of the lung, pleural effusion on the left chest, with liver metastasis, bone metastasis, and brain metastasis. A large amount of pleural effusion reoccurred after many application of extraction and chemotherapy. However, her pleural effusion completely disappeared one month after she was treated with recombinant p53 adenovirus.

### Experiment 5:

### The clinical effects (thyroid gland cancer) of recombinant p53 adenovirus:

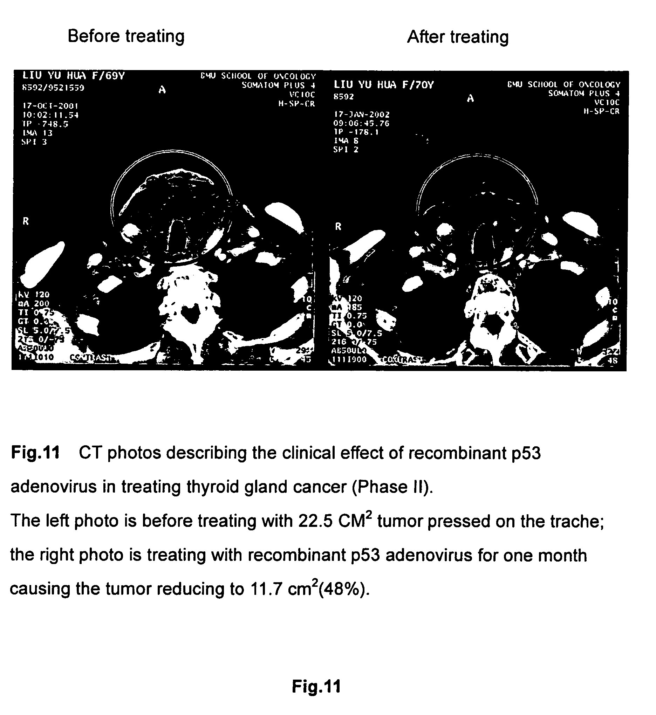

Figure 11 shows the treatment effects of the recombinant p53 adenovirus on thyroid gland cancer in phase II clinical trials. The female patient in this experiment had thyroid gland cancer, reoccurring 1 year after surgery. She had lymph metastasis on the right upper neck which was diagnosed as low-level differentiation squamous cell cancer. No positive effect was evident after 1 year of radiotherapy, chemotherapy and hyperthermia, and she started to have difficulty breathing since the tumor pressed on the trachea. The tumor significantly decreased (by 48%) after the patient was injected 8 times with recombinant p53 adenovirus.

### Experiment 6:

### The clinical effects (carcinoma of the cervix) of recombinant p53 adenovirus:

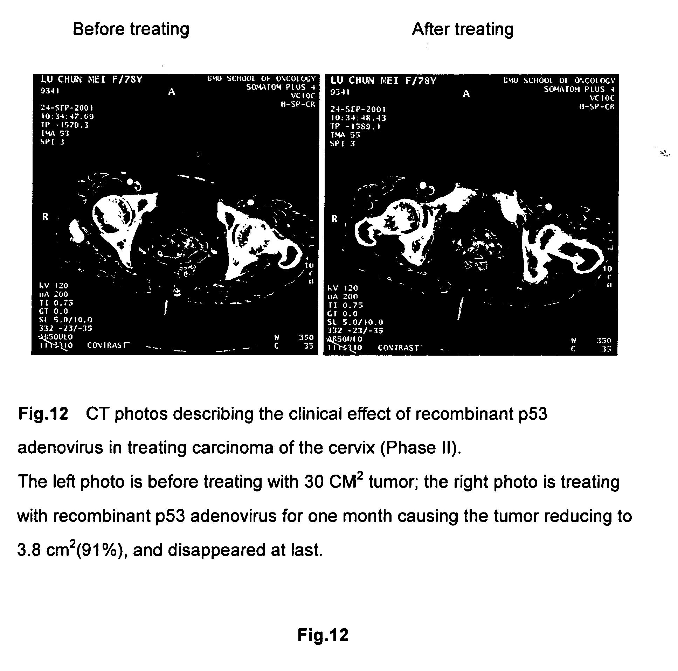

Figure 12 shows the treatment effects of the recombinant p53 adenovirus on carcinoma of the cervix in phase II clinical trials. The carcinoma patient for this experiment had been pathologically diagnosed as squamous cell cancer. The tumor significantly decreased (by 91%) after the patient was injected 8 times with recombinant p53 adenovirus. The tumor completely disappeared after 3 months.

### Experiment 7:

### The clinical effects (metastasis of esophagus cancer) of recombinant p53 adenovirus:

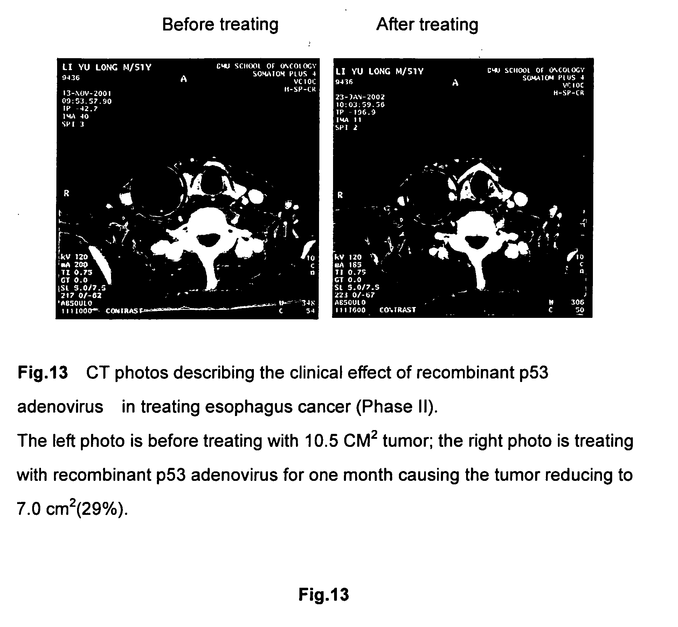

Figure 13 shows the treatment effects of the recombinant p53 adenovirus on the metastasis of esophagus cancer in phase II clinical trials. The patient with nodi lymphatici supraclaviculars metastasis of esophagus cancer in this experiment had been pathologically diagnosed as squamous cell cancer. No effect was evident after radiotherapy. The tumor significantly decreased (by 29%) after the patient was injected 8 times with recombinant p53 adenovirus.

## Claims

1. A recombinant virus, wherein the recombinant is constructed with a viral vector and a human tumor suppressor gene expression cassette by DNA cloning technology, amplifying and propagating in specific genetically engineered cells and expressing the tumor suppressor protein in eukaryotic cells.

2. The recombinant of claim 1, wherein the vector is DNA virus or RNA virus.

3. The recombinant of claim 2, wherein the vector is adenovirus vector or combined vector containing adenovirus vector sequence.

4. The recombinant of claim 1, wherein the said human tumor suppressor gene can be any one of the tumor suppressor genes.

5. The recombinant of claim 4, wherein the human tumor suppressor gene is p53.

6. The recombinant of claim 1, wherein the recombinant containing adenovirus vector and p53 gene, has the following sequence: 
The right end of adenovirus 5
-ATGTTTACCGCCACACTCGCAGGGTCTGCACCTGGTGCGGGTCTCATCGTAC
CTCAGCACCTTCCAGATC70TCTGACATGCGATGTCGACTCGACTGCTTCGCG
ATGTACGGGCCAGATATACGCGTATCTGAGGGGACTAGGGTGTGTTTAGGCG
AAAAGCGGGGCTTCGGTTGTACGCGGTTAGGAGTCCCCTCAGGATATAGTAG
TTTCGCTTTTGCATAGGGAGGGGGAAATGTAGTCTTATGCAATACTCTTGTAG
TCTTGCAACATGGTAACGATGAGTTAGCAACATGCCTTACAAGGAGAGAAAAA
GCACCGTGCATGCCGATTGGTGGAAGTAAGGTGGTACGATCGTGCCTTATTA
GGAAGGCAACAGACGGGTCTGACATGGATTGGACGAACCACTGAATTCCGCA
TTGCAGAGATATTGTATTTAAGTGCCTAGCTCGATACAATAAACGCCATTTGAC
CATTCACCACATTGGTGTGCACCTCCAAGCTTGGTACCGAGCTCGGATCCCG5
23CTAGAGCCACCGTCCAGGGAGCAGGTAGCTGCTGGGCTCCGGGGACACTT
TGCGTTCGGGCTGGGAGCGTCTTTCCACGACGGTGACACGCTTCCCTGGATT
GGCAGCCAGACTGCTTTCCGGGTCACTGCC655ATGGAGGAGCCGCAGTCAGA
TCCTAGCGTCGAGCCCCCTCTGAGTCAGGAAACATTTTCAGACCTATGGAAAC
TACTTCCTGAAAACAACGTTCTGTCCCCCTTGCCGTCCCAAGCAATGGATGAT
TTGATGCTGTCCCCGGACGATATTGAACAATGGTTCACTGAAGACCCAGGTC
CAGATGAAGCTCCCAGAATGCCAGAGGCTGCTCCCCCCGTGGCCCCTGCAC
 
CAGCAGCTCCTACACCGGCGGCCCCTGCACCAGCCCCCTCCTGGCCCCTGT
CATCTTCTGTCCCTTCCCAGAAAACCTACCAGGGCAGCTACGGTTTCCGTCTG
GGCTTCTTGCATTCTGGGACAGCCAAGTCTGTGACTTGCACGTACTCCCCTG
CCCTCAACAAGATGTTTTGCCAACTGGCCAAGACCTGCCCTGTGCAGCTGTG
GGTTGATTCCACACCCCCGCCCGGCACCCGCGTCCGCGCCATGGCCATCTA
CAAGCAGTCACAGCACATGACGGAGGTTGTGAGGCGCTGCCCCCACCATGA
GCGCTGCTCAGATAGCGATGGTCTGGCCCCTCCTCAGCATCTTATCCGAGTG
GAAGGAAATTTGCGTGTGGAGTATTTGGATGACAGAAACACTTTTCGACATAG
TGTGGTGGTGCCCTATGAGCCGCCTGAGGTTGGCTCTGACTGTACCACCATC
CACTACAACTACATGTGTAACAGTTCCTGCATGGGCGGCATGAACCGGAGGC
CCATCCTCACCATCATCACACTGGAAGACTCCAGTGGTAATCTACTGGGACG
GAACAGCTTTGAGGTGCGTGTTTGTGCCTGTCCTGGGAGAGACCGGCGCACA
GAGGAAGAGAATCTCCGCAAGAAAGGGGAGCCTCACCACGAGCTGCCCCCA
GGGAGCACTAAGCGAGCACTGCCCAACAACACCAGCTCCTCTCCCCAGCCAA
AGAAGAAACCACTGGATGGAGAATATTTCACCCTTCAGATCCGTGGGCGTGA
GCGCTTCGAGATGTTCCGAGAGCTGAATGAGGCCTTGGAACTCAAGGATGCC
CAGGCTGGGAAGGAGCCAGGGGGGAGCAGGGCTCACTCCAGCCACCTGAA
GTCCAAAAAGGGTCAGTCTACCTCCCGCCATAAAAAACTCATGTTCAAGACAG
AAGGGCCTGACTCAGACTGA1837CATTCTCCACTTCTTGTTCCCCACTGACAGC
CTCCCACCCCCATCTCTCCCTCCCCTGCCATTTTGGGTTTTGGGTCTTTGAAC
CCTTGCTTGCAATAGGTGTGCGTCAGAAGCACCCAGGACTTCCATTTGCTTTG
TCCCGGGGCTCCACTGAACAAGTTGGCCTGCACTGGTGTTTTGTTGTGGGGA
GGAGGATGGGGAGTAGGACATACCAGCTTAGATTTTAAGGTTTTTACTGTGAG
GGATGTTTGGGAGATGTAAGAAATGTTCTTGCAGTTAAGGGTTAGTTTACAAT
CAGCCACATTCTAGGTAGGGGCCACTTCACCGTACTAACCAGGGAAGCTGTC
CCTCACTGTTGAATTTTCTCTAACTTCAAGGCCCATATCTGTGAAATGCTGGAT
TTGCCCTACCTCGGAATGCTGGCATTTGCACCTACCTCACAGAGTGCATTGTG
AGGGTT2297AATGAAATAATGTACATCTGGCCTTGAAACCACCTTTTATTACATG
GGGTCTAGCGGGATCCACTAGTAACGCCGCCAGTGTGCTGGAATTCTGCAGA
TATCCATCACACTGGCGGCCGCTCGAGCATGCATCTAGAGCTCGCTGATCAG
CCTCGACTGTGCCTTCTAGTTGCCAGCCATCTGTTGTTTGCCCCTCCCCCGTG
CCTTCCTTGACCCTGGAAGGTGCCACTCCCACTGTCCTTTCCTAATAAAATGA
GGAAATTGCATCGCATTGTCTGAGTAGGTGTCATTCTATTCTGGGGGGTGGG
GTGGGGCAGGACAGCAAGGGGGAGGATTGGGAAGACAATAGCAGGCATGCT
GGGGATGCGGTGGGCTCTATGGCTTCTGAGGCGGAAAGAACCAGCTGGGGC
TCGAGGGGGGATCCCCACGCTAGAGCT2733GACTATAATAATAAAACGCCAACT
TTGACCCGGAACGCGGAAAACACCTGAGAAAAACACCTGGGCGAGTCTCCAC
 
GTAAACGGTCAAAGTCCCCGCGGCCCTAGACAAATATTA 2848-the left end of
adenovirus 5
 In which:
1) the right end of adenovirus 5 and the left end of adenovirus 5 are described in the full sequence of adenovirus 5 (Genbank No: NC_001406)
2) 1-70: the right arm of adenovirus (the 70^{th} base locates at adenovirus gene sequence 3328)
3) 71-523: Rous Sarcoma Virus (RSV) LTR (promoter)
4) 524-655: 5' end non-translating region
5) 656-1837: p53 gene coding sequence
6) 1838-2733: 3' end non-translating region (poly Adenosine tail starting at 2298)
7) 2734-2848: the left arm of adenovirus (base at 2734 is positioned at 452 of adenovirus 5 gene sequence)

7. The recombinant of claim 1, wherein the gene expression cassette of the recombinant is a specific sequence composed of promoter-p53cDNA-poly adenosine.

8. The recombinant of claim 7, wherein the upstream of the gene expression cassette is any eukaryotic cell promoters, prokaryotic cell promoters or virus promoters, and the downstream is any of the eukaryotic gene poly adenosine residues (Poly A tail).

9. The recombinant of claim 1, wherein the recombinant is obtained in prokaryotic cells by homologous recombination, including:
1) the recombinant pGT-2 is obtained by homologous recombination of adenovirus and plasmid pGT-1 (containing two inverted terminal repeats on both ends of adenovirus) in *E. coli;*
2) the recombinant pGT-3 is obtained by homologous recombination of pGT-2 and artificial sequence "the right arm of adenovirus/ promoter-p53cDNA-poly A / the left arm of adenovirus "in *E*. *coli;*
3) the recombinant p53 adenovirus is obtained by discarding the prokaryotic sequence using endonuclease *Pac*l*.*

10. The recombinant of claim 9, wherein the recombinant is obtained in any prokaryotic cells by homologous recombination.

11. The recombinant of claim 1 is used to prepare medicines for treating malignant tumors.

12. The recombinant of claim 1 is used to prepare medicines for preventing tumors or post-surgery recurrence of tumors.

13. The recombinant of claim 1 is used to prepare medicines for intravenous injection, artery injection, intratumoral injection, intramuscular injection, subcutaneous injection, organ injection and injection in pleural effusion or ascites.


Sequence

<110> PENG, Zhaohui etc.

<120> A Recombinant Constructed by a Virus Vector and a Human Tumor Suppressor Gene and
Its Use

<130> CPS41236

<140> PCT/CN2004/000465

<141> 2004-03-08

<150> CN03165032.5

<151> 2003-03-06

<160> 1

<170> PatentIn version 3.1

<210> 1

<211> 2847

<212> DNA

<213> Artifical sequence

<400> 1
atgtttaccg ccacactcgc agggtctgca cctggtgcgg gtctcatcgt acctcagcac 60

cttccagatc tctgacatgc gatgtcgact cgactgcttc gcgatgtacg ggccagatat 120

acgcgtatct gaggggacta gggtgtgttt aggcgaaaag cggggcttcg gttgtacgcg 180

gttaggagtc ccctcaggat atagtagttt cgcttttgca tagggagggg gaaatgtagt 240

cttatgcaat actcttgtag tcttgcaaca tggtaacgat gagttagcaa catgccttac 300

aaggagagaa aaagcaccgt gcatgccgat tggtggaagt aaggtggtac gatcgtgcct 360

tattaggaag gcaacagacg ggtctgacat ggattggacg aaccactgaa ttccgcattg 420

cagagatatt gtatttaagt gcctagctcg atacaataaa cgccatttga ccattcacca 480

cattggtgtg cacctccaag cttggtaccg agctcggatc ccgctagagc caccgtccag 540

ggagcaggta gctgctgggc tccggggaca ctttgcgttc gggctgggag cgtctttcca 600

cgacggtgac acgcttccct ggattggcag ccagactgct ttccgggtca ctgccatgga 660

ggagccgcag tcagatccta gcgtcgagcc ccctctgagt caggaaacat tttcagacct 720

atggaaacta cttcctgaaa acaacgttct gtcccccttg ccgtcccaag caatggatga 780

tttgatgctg tccccggacg atattgaaca atggttcact gaagacccag gtccagatga 840

agctcccaga atgccagagg ctgctccccc cgtggcccct gcaccagcag ctcctacacc 900

ggcggcccct gcaccagccc cctcctggcc cctgtcatct tctgtccctt cccagaaaac 960

ctaccagggc agctacggtt tccgtctggg cttcttgcat tctgggacag ccaagtctgt 1020

gacttgcacg tactcccctg ccctcaacaa gatgttttgc caactggcca agacctgccc 1080

tgtgcagctg tgggttgatt ccacaccccc gcccggcacc cgcgtccgcg ccatggccat 1140

ctacaagcag tcacagcaca tgacggaggt tgtgaggcgc tgcccccacc atgagcgctg 1200

ctcagatagc gatggtctgg cccctcctca gcatcttatc cgagtggaag gaaatttgcg 1260

tgtggagtat ttggatgaca gaaacacttt tcgacatagt gtggtggtgc cctatgagcc 1320

gcctgaggtt ggctctgact gtaccaccat ccactacaac tacatgtgta acagttcctg 1380

catgggcggc atgaaccgga ggcccatcct caccatcatc acactggaag actccagtgg 1440

taatctactg ggacggaaca gctttgaggt gcgtgtttgt gcctgtcctg ggagagaccg 1500

gcgcacagag gaagagaatc tccgcaagaa aggggagcct caccacgagc tgcccccagg 1560

gagcactaag cgagcactgc ccaacaacac cagctcctct ccccagccaa agaagaaacc 1620

actggatgga gaatatttca cccttcagat ccgtgggcgt gagcgcttcg agatgttccg 1680

agagctgaat gaggccttgg aactcaagga tgcccaggct gggaaggagc caggggggag 1740